# EUROPEAN PATENT SPECIFICATION

(11) **EP 4 268 183 B1**
(45) Date of publication and mention of the grant of the patent: **27.08.2025**
(21) Application number: 21839883.2
(22) Date of filing: 15.12.2021
(51) Int. Cl.: G06T 7/00, A61B 6/00, A61B 6/03, A61B 6/50

(54) **LOCATING VASCULAR CONSTRICTIONS**
LOKALISIERUNG VON GEFÄSSVERENGUNGEN
LOCALISATION DE CONSTRICTIONS VASCULAIRES

(30) Priority: 22.12.2020 US 202063129292 P
(43) Date of publication of application: 01.11.2023
(73) Proprietor: Koninklijke Philips N.V., 5656 AG Eindhoven (NL)
(72) Inventor: ERKAMP, Ramon, Quido, 5656 AE Eindhoven (NL); SINHA, Ayushi, 5656 AE Eindhoven (NL); SALEHI, Leili, 5656 AE Eindhoven (NL); PANSE, Ashish, Sattyavrat, 5656 AE Eindhoven (NL); TOPOREK, Grzegorz, Andrzej, 5656 AE Eindhoven (NL)
(74) Representative: Philips Intellectual Property & Standards
(86) International application number: PCT/EP2021/085820
(87) International publication number: WO 2022/136043

(56) References cited:
- EP-A1- 0 635 805
- CN-A- 111 833 343
- US-A1- 2011 081 057
- CONG CHAO ET AL: "Automated Stenosis Detection and Classification in X-ray Angiography Using Deep Neural Network", 2019 IEEE INTERNATIONAL CONFERENCE ON BIOINFORMATICS AND BIOMEDICINE (BIBM), IEEE, 18 November 2019 (2019-11-18), pages 1301 - 1308, XP033703872, DOI: 10.1109/BIBM47256.2019.8983033
- MAJD ZREIK ET AL: "A Recurrent CNN for Automatic Detection and Classification of Coronary Artery Plaque and Stenosis in Coronary CT Angiography", ARXIV.ORG, CORNELL UNIVERSITY LIBRARY, 201 OLIN LIBRARY CORNELL UNIVERSITY ITHACA, NY 14853, 12 April 2018 (2018-04-12), XP080997592, DOI: 10.1109/TMI.2018.2883807

## Description

### TECHNICAL FIELD

The present disclosure relates to locating a vascular constriction in a temporal sequence of angiographic images. A computer-implemented method, a processing arrangement, a system, and a computer program product, are disclosed.

### BACKGROUND

Vascular constrictions such as thromboses and stenoses limit the supply of blood within the vasculature. Without treatment, vascular constrictions can lead to serious medical consequences. For example, ischemic stroke is caused by a blockage of the vasculature in the brain. Stroke occurs rapidly and requires immediate treatment in order to minimize the amount of damage to the brain.

An important step in determining how to treat a suspected vascular constriction is to identify its location. This is often performed using angiographic images. For example, an article "Automated stenosis detection and classification in X-Ray angiography using deep neural network" by Chao Cong et al., 2019 IEEE International Conference on Bioinformatics and Biomedicine (BIBM), IEEE, 18 November 2019, pages 1301-1308 (XP033703872) describes a deep-learning based workflow for localization and severity classification of stenosis on coronary angiography images.

Further, in the case of a suspected stroke, an initial computed tomography "CT" angiogram may be performed on the brain to try to identify the location of a suspected vascular constriction. However, the small size of vascular constrictions hampers this determination from the CT angiogram. Subsequently, a CT perfusion scan may be performed. A CT perfusion scan indicates regions of the brain that are not receiving sufficient blood flow. However, a CT perfusion scan typically only identifies a section of the brain that may be affected by the vascular constriction, rather than a specific branch of the vasculature. Subsequently, a contrast agent may be injected into the vasculature and a fluoroscopy scan may be performed on the brain to try to identify the stenotic region. The fluoroscopy scan provides a video sequence of angiographic images representing a flow of a contrast agent within the vasculature. However, locating a vascular constriction within the angiographic images is time-consuming. A radiologist may try to identify a region with interrupted flow by repeatedly zooming-in to view individual branches of the vasculature, and then zooming-out again whilst following the course of the vasculature. A small vascular constriction may easily be overlooked in this process, potentially delaying a vital intervention.

Consequently, there is a need for improvements in determining the location of vascular constrictions in angiographic images.

### SUMMARY

According to one aspect of the present disclosure, a computer-implemented method of locating a vascular constriction in a temporal sequence of angiographic images, is provided. The method includes:
receiving a temporal sequence of angiographic images representing a flow of a contrast agent within a vasculature;
for at least some of the angiographic images in the temporal sequence, computing a differential image representing a difference in image intensity values between a current image and an earlier image in the sequence in a plurality of sub-regions of the vasculature;
identifying, from the differential images, temporal sequences of a subset of the sub-regions and in which subset the contrast agent enters the sub-region, and the contrast agent subsequently leaves the sub-region;
inputting the identified temporal sequences of the subset into a neural network trained to classify, from temporal sequences of angiographic images of the vasculature, a sub-region of the vasculature as including a vascular constriction; and
identifying, a sub-region that includes the vascular constriction based on the classification provided by the neural network.

According to another aspect of the present disclosure, a computer implemented method of training a neural network to locate a vascular constriction in a temporal sequence of angiographic images, is provided. The method includes:
receiving angiographic image training data including a plurality of temporal sequences of angiographic images representing a flow of contrast agent within a plurality of sub-regions of a vasculature; each temporal sequence being classified with a ground truth classification as including a vascular constriction or classified as not including a vascular constriction;
inputting the received angiographic image training data into the neural network; and adjusting parameters of the neural network based on a difference between the classification of each inputted temporal sequence generated by the neural network, and the ground truth classification.

Further aspects, features and advantages of the present disclosure will become apparent from the following description of examples, which is made with reference to the accompanying drawings.

### BRIEF DESCRIPTION OF THE DRAWINGS

Fig. 1 illustrates a temporal sequence of angiographic images 110.
Fig. 2 illustrates a temporal sequence of angiographic images 110 including a vascular constriction 140.
Fig. 3 is a flowchart illustrating a method of locating a vascular constriction in a temporal sequence of angiographic images, in accordance with some aspects of the disclosure.
Fig. 4 is a schematic diagram illustrating a method of locating a vascular constriction in a temporal sequence of angiographic images, in accordance with some aspects of the disclosure.
Fig. 5 illustrates a temporal sequence of differential images for a sub-region 120_{i,j}, in accordance with some aspects of the disclosure and wherein the differential images are generated using a mask image as the earlier image in the sequence.
Fig. 6 illustrates a temporal sequence of differential images for a sub-region 120_{i,j} within a time interval between the contrast agent entering the sub-region, and the contrast agent leaving the sub-region, in accordance with some aspects of the disclosure.
Fig. 7 is a schematic diagram illustrating a display 200 indicating a sub-region 120_{i,j} that includes a vascular constriction 140, in accordance with some aspects of the disclosure.
Fig. 8 is a flowchart illustrating a method of training a neural network to locate a vascular constriction in a temporal sequence of angiographic images, in accordance with some aspects of the disclosure.
Fig. 9 is a schematic diagram illustrating a system 300 for locating a vascular constriction in a temporal sequence of angiographic images, in accordance with some aspects of the disclosure.

### DETAILED DESCRIPTION

Examples of the present disclosure are provided with reference to the following description and the figures. In this description, for the purposes of explanation, numerous specific details of certain examples are set forth. Reference in the specification to "an example", "an implementation" or similar language means that a feature, structure, or characteristic described in connection with the example is included in at least that one example. It is also to be appreciated that features described in relation to one example may also be used in another example, and that all features are not necessarily duplicated in each example for the sake of brevity. For instance, features described in relation to a computer-implemented method may be implemented in a processing arrangement, and in a system, and in a computer program product, in a corresponding manner.

In the following description, reference is made to computer implemented methods that involve locating a vascular constriction in a temporal sequence of angiographic images. Reference is made to a vascular constriction in the form of a stenosis, i.e. a narrowing or constriction to the body or opening of a vessel conduit. The stenosis may have various origins. By way of an example, the stenosis may be caused by atherosclerosis, which causes fatty deposits and a buildup of plaque in the blood vessels, and may lead to ischemic stroke. By way of another example, the stenosis may be caused by a thrombus, wherein a blood clot develops in a blood vessel and reduces the flow of blood through the vessel. However, it is to be appreciated that the methods disclosed herein are not limited to these examples and may also be used to locate other types of vascular constrictions, and that these may have various underlying causes. Reference is also made to examples of vascular constrictions in the brain. However, it is also to be appreciated that the methods disclosed herein may also be used to locate vascular constrictions in other regions of the body, such as in the heart, the leg, and so forth. Thus, it is to be appreciated that the methods disclosed herein may be used to locate vascular constrictions in general.

It is noted that the computer-implemented methods disclosed herein may be provided as a non-transitory computer-readable storage medium including computer-readable instructions stored thereon which, when executed by at least one processor, cause the at least one processor to perform the method. In other words, the computer-implemented methods may be implemented in a computer program product. The computer program product can be provided by dedicated hardware or hardware capable of running the software in association with appropriate software. When provided by a processor, or "processing arrangement", the functions of the method features can be provided by a single dedicated processor, by a single shared processor, or by a plurality of individual processors, some of which can be shared. The explicit use of the terms "processor" or "controller" should not be interpreted as exclusively referring to hardware capable of running software, and can implicitly include, but is not limited to, digital signal processor "DSP" hardware, read only memory "ROM" for storing software, random access memory "RAM", a non-volatile storage device, and the like. Furthermore, examples of the present disclosure can take the form of a computer program product accessible from a computer usable storage medium or a computer-readable storage medium, the computer program product providing program code for use by or in connection with a computer or any instruction execution system. For the purposes of this description, a computer-usable storage medium or computer-readable storage medium can be any apparatus that can comprise, store, communicate, propagate, or transport a program for use by or in connection with an instruction execution system, apparatus, or device. The medium can be an electronic, magnetic, optical, electromagnetic, infrared, or semiconductor system or device or propagation medium. Examples of computer-readable media include semiconductor or solid-state memories, magnetic tape, removable computer disks, random access memory "RAM", read only memory "ROM", rigid magnetic disks, and optical disks. Current examples of optical disks include compact disk-read only memory "CD-ROM", optical disk-read/write "CD-R/W", Blu-Ray^{™}, and DVD.

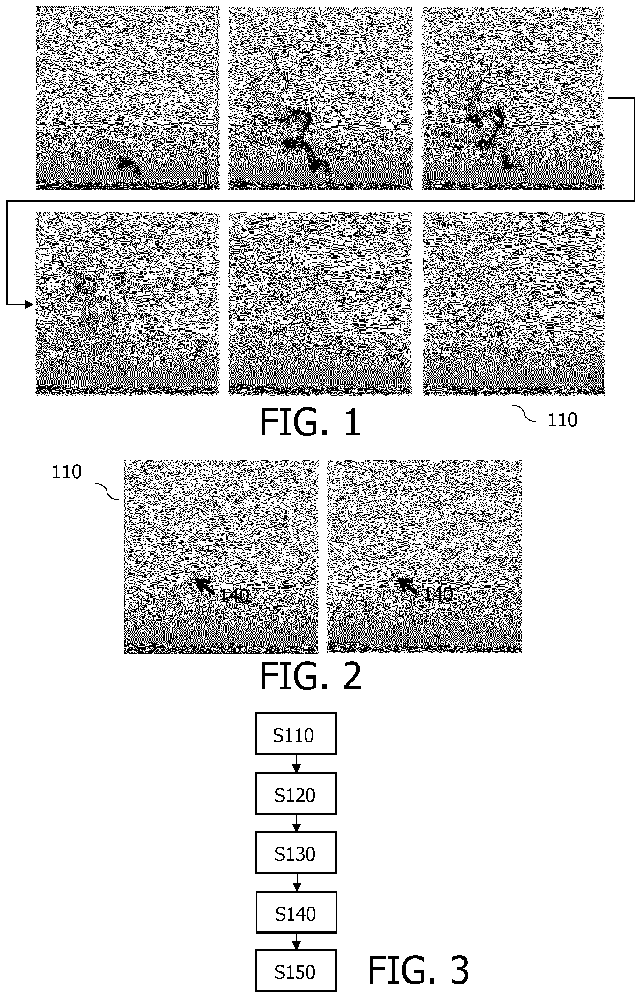

Fig. 1 illustrates a temporal sequence of angiographic images 110. The images in Fig. 1 are generated by a fluoroscopy, or live X-ray, imaging procedure that is performed on the brain to try to identify a stenotic region in the event of a suspected ischemic stroke. The angiographic images in Fig. 1, are obtained after a radiopaque contrast agent has been injected into the vasculature, and therefore represent a flow of a contrast agent within the vasculature, in particular in the brain.

As may be appreciated, locating a vascular constriction within the angiographic images in Fig. 1 can be time-consuming. A radiologist may try to identify a region with interrupted flow by repeatedly zooming-in to view individual branches of the vasculature, and then zooming-out again. Ultimately the vascular constriction may be found in one of the branches, as illustrated in Fig. 2, which illustrates a temporal sequence of angiographic images 110 including a vascular constriction 140. A small vascular constriction may easily be overlooked in this process, potentially delaying a vital intervention.

The inventors have determined an improved method of locating a vascular constriction in a temporal sequence of angiographic images. Thereto, Fig. 3 is a flowchart illustrating a method of locating a vascular constriction in a temporal sequence of angiographic images, in accordance with some aspects of the disclosure. With reference to Fig. 3, the method includes:
receiving S110 a temporal sequence of angiographic images 110 representing a flow of a contrast agent within a vasculature;
for at least some of the angiographic images in the temporal sequence, computing S120 a differential image representing a difference in image intensity values between a current image and an earlier image in the sequence in a plurality of sub-regions 120_{i,j} of the vasculature;
identifying S130, from the differential images, temporal sequences of a subset of the sub-regions 120_{i,j} and in which subset the contrast agent enters the sub-region, and the contrast agent subsequently leaves the sub-region;
inputting S140 the identified temporal sequences of the subset into a neural network 130 trained to classify, from temporal sequences of angiographic images of the vasculature, a sub-region 120_{i,j} of the vasculature as including a vascular constriction 140; and
identifying S150, a sub-region that includes the vascular constriction 140 based on the classification provided by the neural network 130.

With reference to Fig. 3, the temporal sequence of angiographic images 110 received in operation S110 are two-dimensional, i.e. "projection" images, although it is also contemplated that these may be 3D images. The temporal sequence of angiographic images 110 may therefore be generated by an X-ray or computed tomography "CT" imaging system. The temporal sequence of angiographic images 110 may be the result of a fluoroscopy imaging procedure performed on the vasculature in the brain, as illustrated in Fig. 2, or on another part of the body. The temporal sequence of angiographic images 110 received in operation S110 may be received from various sources, including from an X-ray or CT imaging system, from a database, from a computer readable storage medium, from the cloud, and so forth. The data may be received using any form of data communication, such as wired or wireless data communication, and may be via the internet, an ethernet, or by transferring the data by means of a portable computer-readable storage medium such as a USB memory device, an optical or magnetic disk, and so forth.

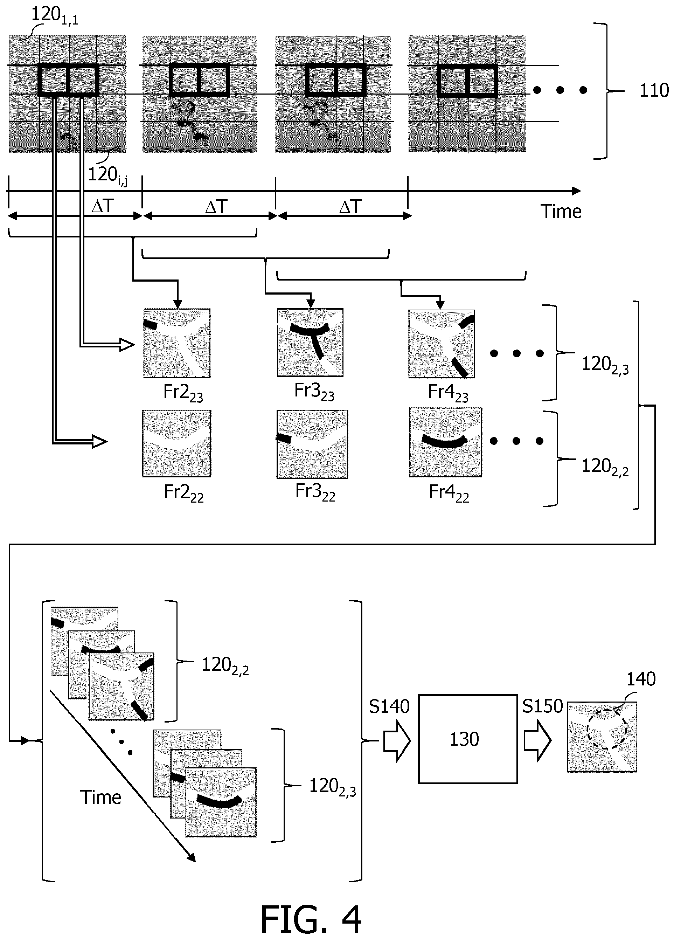

With continued reference to Fig. 3, in operation S120 a differential image representing a difference in image intensity values between a current image and an earlier image in the sequence, is computed for at least some of the angiographic images in the temporal sequence, in a plurality of sub-regions 120_{i,j} of the vasculature. Operation S120 is described with reference to Fig. 4, which is a schematic diagram illustrating a method of locating a vascular constriction in a temporal sequence of angiographic images, in accordance with some aspects of the disclosure. With reference to the upper portion of Fig. 4, a temporal sequence of angiographic images 110 are generated with a period ΔT, in sub-regions 120_{i,j} of the vasculature.

Various techniques are contemplated for computing the differential images in operation S120. In one example, a time period ΔT between the generation of a current image and the generation of an earlier image in the sequence, that is used to compute each differential image, is predetermined. For example, if, as illustrated in Fig. 4, the time period ΔT is equal to the period between successive image frames, each differential image, illustrated in the central portion of Fig. 4 for sub-regions 120_{2,2} and 120_{2,3} represents a rate of change in image intensity values between the current image and the preceding image in the sequence. By way of explanation, contrast agent can be seen to enter sub-region 120_{2,3} in image frame Fr2 and slightly later in time, in image frame Fr3, contrast agent enters sub-region 120_{2,2}. Since these image frames are differential images, the flow into the region is represented by a pulse of contrast agent that progressively travels through the respective branch of the vasculature, rather than by a lengthening dark region of contrast agent - as might be expected in a conventional angiographic image. The time period may be equal to any integer multiple of the period between successive image frames in the temporal sequence, and thus, in a similar manner, each differential image represents a rate of change in image intensity values between the current image and the earlier image in the sequence.

In another example, the earlier image is provided by a mask image, and the same mask image is used to compute each differential image. The mask image is fluoroscopic image that is generated prior to the injection of the contrast agent into the vasculature. In this example, the differential images computed in operation S120 are so-called digital subtraction angiography "DSA" images. In both these examples, image features that are common to the current image and the earlier image, arising for example from bone, are removed from the resulting differential images.

In yet another example a combination of these techniques is used. In this example, the temporal sequence of angiographic images 110 are DSA images, and differential images are computed from the DSA images by subtracting from the image intensity values of a current DSA image, the image intensity values from an earlier DSA image in the sequence. The time period ΔT between the generation of the current DSA image and the generation of the earlier DSA image, that is used to compute each differential image, is again, predetermined. The time period ΔT may be an integer multiple of the period between successive image frames. Here, again each differential image represents a rate of change in image intensity values, in this example the rate of change being between the current DSA image and the earlier DSA image in the sequence.

Various techniques are contemplated for defining the sub-regions 120_{i,j} in operation S120. In one example, the sub-regions 120_{i,j} of the vasculature are defined by dividing the angiographic images in the received temporal sequence into a plurality of predefined sub-regions. This may be achieved by applying a grid of predefined regions to the angiographic images, as illustrated in the upper portion of Fig. 4. Other grids than the example illustrated in Fig. 4 may alternatively be used. For example, the grid may have equal-sized, unequal-sized, regular-shaped, or irregular-shaped sub-regions. The grid may have contiguous or non-contiguous sub-regions. The grid may have a different number of sub-regions to the example illustrated in Fig. 4.

In another example, the sub-regions 120_{i,j} of the vasculature are defined by segmenting the vasculature in the angiographic images and defining a plurality of sub-regions that overlap the vasculature. Various techniques for segmenting the vasculature in angiographic images are known from a document by Moccia, S. et al., entitled Blood vessel segmentation algorithms - Review of methods, datasets and evaluation metrics, Computer Methods and Programs in Biomedicine, Volume 158, May 2018, Pages 71 - 91. In this example, sub-regions may be defined by applying predetermined shapes, such as a rectangle or square, to sections of the vasculature such that the shapes overlap the vasculature. Continuing with this example, branches in the segmented vasculature may be identified, and an amount of contrast agent at positions along an axial length of each branch may be determined. The sub-region may then be represented as a two-dimensional graph indicating the amount contrast agent plotted against the axial length of the branch. In this example, the shape of the vasculature is essentially stretched into a straight line, and the amount of contrast agent along an axial length of each branch determined by computing the amount of contrast agent at positions along e.g. the centerline of the branch.

Returning to Fig. 3, in operation S130, temporal sequences of a subset of the sub-regions 120_{i,j} are identified from the differential images, and in which subset the contrast agent enters the sub-region, and the contrast agent subsequently leaves the sub-region. The inventors have determined that there is significant redundancy in the temporal sequence of angiographic images used by radiologists to try to identify vascular constrictions. The inventors have also recognized that sub-regions in which the contrast agent enters the sub-region, and the contrast agent subsequently leaves the sub-region, contain valuable information that can be used to identify a location of a vascular constriction. Operation S130 identifies this information as a subset of the sub-regions, and in the later operation S140, this subset is inputted into a neural network to identify a sub-region of the vasculature as including a vascular constriction. In so doing, operation S130 may reduce the complexity and/or time taken for the neural network to analyses the angiographic images 110 and to determine a location of a vascular constriction.

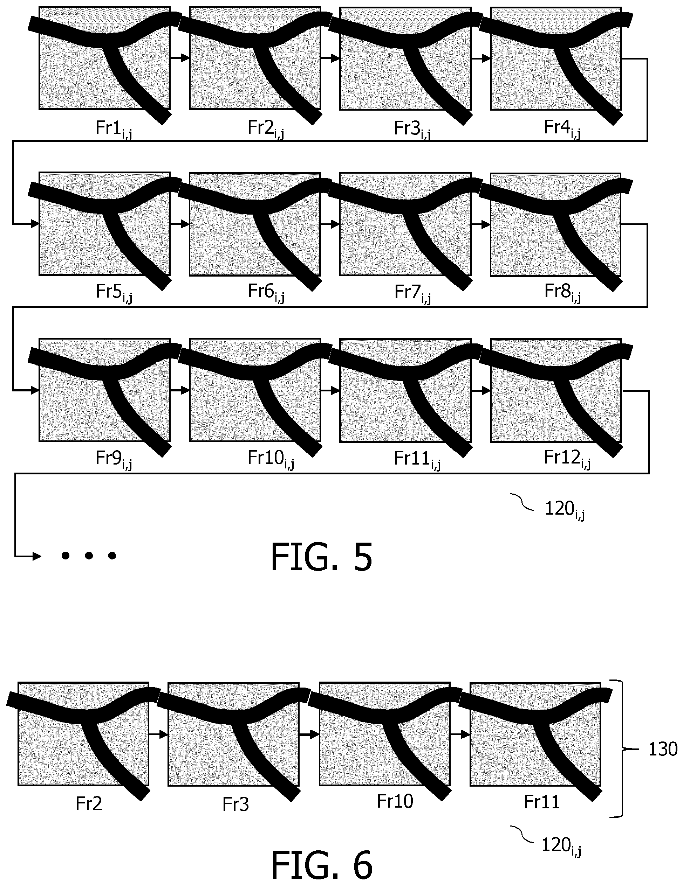

The operation S130 is described further with reference to Fig. 3, Fig. 4, Fig. 5 and Fig. 6. Fig. 5 illustrates a temporal sequence of differential images for a sub-region 120_{i,j}, in accordance with some aspects of the disclosure and wherein the differential images are generated using a mask image as the earlier image in the sequence. The sub-region 120_{i,j} illustrated in Fig. 5 represents a sub-region similar to the sub-regions 120_{2,2} and 120_{2,3} in the central portion of Fig. 4, and which were generated by applying the example grid of predefined regions to the vasculature. In contrast to the differential images illustrated in Fig. 4, since the differential images in Fig. 5 were generated using a mask image as the earlier image in the sequence, the flow of contrast agent in the branches of the vasculature is illustrated as a lengthening dark region rather than a moving pulse, as in Fig. 4. For ease of explanation, the sub-regions in Fig. 5 are identified with row and column indices i and j. In the figures, contrast agent is indicated in black. In the example temporal sequence Fr1..Fr12 for sub-region 120_{i,j} illustrated in Fig. 5, contrast agent does indeed enter the sub-region, and subsequently leave the sub-region. The temporal sequence for the sub-region 120_{i,j} illustrated in Fig. 5 would therefore form part of the subset that are identified in operation S130 and inputted into the neural network in operation S140. The entering, and the leaving of the contrast agent from the sub-region, and also the sub-regions themselves, may be detected by applying a threshold to the contrast agent detected in a branches of the segmented vasculature. By contrast, a temporal sequence (not illustrated) for a sub-region of the vasculature in which the contrast agent does not enter the sub-region, would not form part of the subset that are identified in operation S130, and would therefore not be inputted into the neural network in operation S140.

Returning to the central portion of Fig. 4; in a similar manner, contrast agent can be seen to enter sub-region 120_{2,3} in image frame Fr2, and slightly later in time, in image frame Fr3, contrast agent enters sub-region 120_{2,2}. If the contrast agent in these sub-regions subsequently leaves the respective sub-region, the temporal sequences for these sub-regions would also form part of the subset that are identified in operation S130 and inputted into the neural network in operation S140.

Further redundancy in the temporal sequence of angiographic images used by radiologists to try to identify vascular constrictions, has also been recognized by the inventors, and in some examples, further refinements may be made to the temporal sequences of the subset of the sub-regions 120_{i,j} that are identified in operation S130. These refinements further reduce that amount of data that is inputted into the neural network, and may further reduce the complexity and/or time taken for the neural network to analyses the angiographic images 110 and to determine a location of a vascular constriction.

In one example of these refinements, portions of the temporal sequences are identified. In this example, the operation of identifying S130 temporal sequences of a subset of the sub-regions 120_{i,j}, comprises:
identifying portions Fr2..Fr11 of the temporal sequences generated between the contrast agent entering the sub-region and the contrast agent leaving the sub-region; and wherein the inputting the temporal sequences of the subset into a neural network 130, comprises inputting the identified portions Fr2..Fr11 of the temporal sequences of the subset into the neural network 130.

In this example, and with reference to Fig. 5, the illustrated temporal sequence for example sub-region 120_{i,j} of the vasculature, includes differential image frames Fr1..Fr12 wherein the differential images are generated using a mask image as the earlier image in the sequence. In this example, contrast agent may be seen to enter the example sub-region 120_{i,j} at a time corresponding to differential image frame Fr2, and to leave the sub-region 120_{i,j} at a time corresponding to differential image frame Fr11. In this example, the portion within the time period from Fr2 to Fr11, is identified in the operation S130 and inputted into the neural network in operation S140.

A further refinement may also be made to the portions of the temporal sequences that are identified in operation S130. This is described with reference to Fig. 5, as well as Fig. 6, which illustrates a temporal sequence of differential images for a sub-region 120_{i,j} within a time interval between the contrast agent entering the sub-region, and the contrast agent leaving the sub-region, in accordance with some aspects of the disclosure.

In this example, the operation of identifying S130 temporal sequences of a subset of the sub-regions, may also include:
identifying further portions Fr4..Fr9 of the temporal sequences wherein the sub-region has a maximum amount of contrast agent, and excluding from the identified portions Fr2..Fr11 of the temporal sequences the further portions Fr4..Fr9 of the temporal sequences.

With reference to Fig. 5 and Fig. 6, in this example, a further portion of the temporal sequence for sub-region 120_{i,j} is identified, specifically, at times corresponding to differential image frames Fr4, Fr5, Fr6, Fr7, Fr8 and Fr9, in which the sub-region 120_{i,j} has a maximum amount of contrast agent. In this example, in operation S130, differential image frames Fr4, Fr5, Fr6, Fr7, Fr8 and Fr9 are excluded from the portions Fr2..Fr11 of the temporal sequences that are inputted into the neural network in operation S140. Thus, as illustrated in Fig. 6, in this example, image frames Fr2, Fr3, Fr10 and Fr11 are selected from the image frames illustrated in Fig. 5, are inputted into the neural network. By excluding from the temporal sequences that are inputted into the neural network, portions of the temporal sequences wherein the sub-region has a maximum amount of contrast agent, the amount of data inputted into the neural network is further reduced. Moreover, these excluded portions do not significantly contribute to the neural network's ability to determine the location of a vascular constriction. Consequently, this may further reduce the complexity and/or time taken for the neural network to analyses the angiographic images 110, without degrading the accuracy of the neural network's determination.

Returning to the method illustrated in the flowchart of Fig. 3, having identified the temporal sequences of the subset of the sub-regions 120_{i,j} in operation S130, in operation S140, these are inputted into a neural network 130 that is trained to classify, from temporal sequences of angiographic images of the vasculature, a sub-region 120_{i,j} of the vasculature as including a vascular constriction 140. The operation S140 is illustrated in the lower portion of Fig. 4, and in some examples may include stacking, i.e. arranging, in the time domain, the identified temporal sequences of the subset of the sub-regions, prior to inputting the identified temporal sequences of the subset into the neural network 130.

As also illustrated in Fig. 4, after the inputting in operation S140, the operation S150 is performed. The operation S150 includes identifying S150, a sub-region that includes the vascular constriction 140 based on the classification provided by the neural network 130.

A variety of techniques are contemplated for use in identifying a sub-region that includes the vascular constriction in the operation S150. These may for example include identifying the sub-region on a display. In one example, an image of the sub-region may be provided wherein the location is identified by means of overlaying a shape, as illustrated by the example dashed circle in Fig. 4. In another example, an image of the sub-region may be color-coded to identify the location, or another identifier such as an arrow may be used to identify the location. In another example, the identifying S150, a sub-region that includes the vascular constriction 140, includes:
displaying a temporal sequence of angiographic images 110 representing a flow of a contrast agent within the identified sub-region that includes the vascular constriction 140, or displaying a temporal sequence of differential images representing a flow of a contrast agent within the identified sub-region that includes the vascular constriction 140.

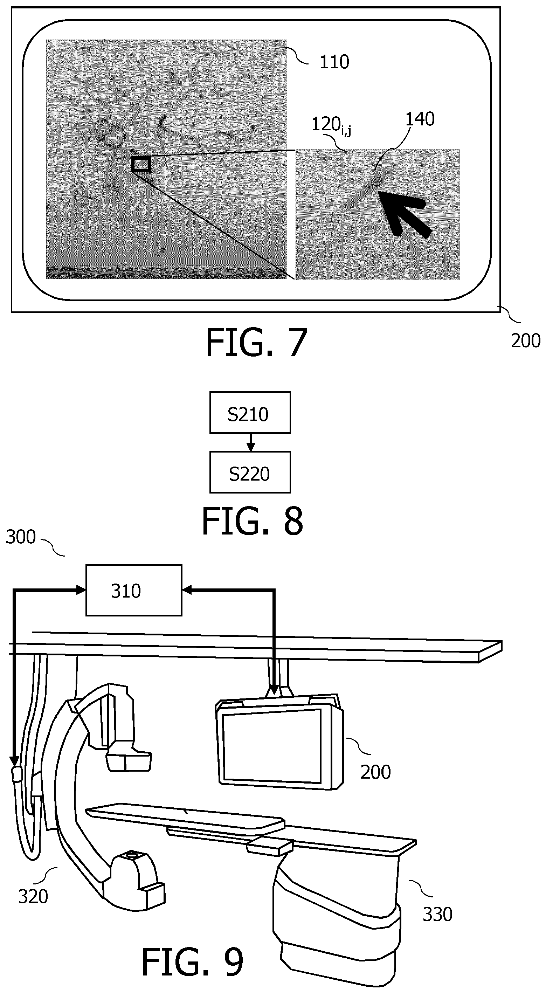

In this example, the displayed temporal sequence may be provided on a display for the identified sub-region alone, thereby permitting a user to focus attention on this sub-region, or for all sub-regions. In some examples, the displayed temporal sequence may be provided for the identified sub-region, as well as for all sub-regions. The displayed temporal sequences may correspond in time to one another, the temporal sequence for all sub-regions providing a large field of view and the temporal sequence for the identified sub-region providing a small field of view. This is illustrated in Fig. 7, which is a schematic diagram illustrating a display 200 indicating a sub-region 120_{i,j} that includes a vascular constriction 140, in accordance with some aspects of the disclosure. The displayed temporal sequences may optionally be displayed for a time interval between the contrast agent entering the sub-region and the contrast agent leaving the sub-region; or for a portion of this time interval, for example by omitting image frames wherein the sub-region has a maximum amount of contrast agent in the sub-region, and thus only displaying image frames showing the contrast agent entering the sub-region and image frames showing the contrast agent leaving the sub-region.

In yet another example, the neural network 130 is trained to generate a probability score of the sub-region 120_{i,j} of the vasculature including a vascular constriction 140. The neural network 130 may include a regression-type neural network or classification-type network for this purpose. The probability score may be identified in the operation S150. For example, an angiographic image, or a temporal sequence of angiographic images, may be displayed, indicating the probability scores of one or more of the sub-regions. This may for example be indicated with a color-coded frame around the sub-region, or by color-coding the sub-region of the vasculature. For example, regions with a relatively high probability values may be highlighted in red, and regions with relatively low probability values may be highlighted in green. Regions with intermediate probability values may be highlighted in orange.

The use of various types of neural network 130 to provide the functionality described in the above methods, is contemplated. Various types of classification neural network may be used, including a convolutional neural network "CNN", a recurrent neural network "RNN", such as for example a Long Short Term Memory "LSTM", a temporal convolutional network "TCN", a transformer, a multi-layer perceptron, a decision-tree such as for example random forest, multivariate regression (e.g. logistic regression), or a combination thereof.

In one example the neural network 130 includes a CNN, and the temporal sequences of the subset of the sub-regions 120_{i,j} that are identified from the differential images in operation S130 are stacked, i.e. arranged, in the time dimension, with each frame as a separate channel, and this 3D data input, is inputted into the a CNN. As an alternative to using 2D filters, a CNN could include 3D filters i.e. 3D convolution kernels. Alternatively, these sequences could be initially processed by a CNN and a low-dimensional representation i.e. feature space is inputted in a sequential frame after frame manner into a RNN where each frame forms a directed graph along the temporal sequence. In an RNN the output of a current frame may be dependent on one or more previous frames. The network may include a uni- or bi-directional long short-term memory "LSTM" architecture. It is noted that the directionality of flow in the images is less important that the speed of flow in the images. In order to account for different numbers of image frames in each sequence, shorter sequences may be padded with empty images at the beginning or at the end of the sequence. Alternatively, interpolation may be used to interpolate new image frames in order to equalize the number of frames in all temporal sequences. The frame rate may be included as a parameter of the neural network.

In another example, a fully convolutional neural network may be used to provide neural network 130. Feature maps may be used to compensate for differences in inputted sequence length. In order to increase the accuracy of this neural network, during training, the inputted sequences may be resized by, for instance, by randomly dropping or interpolating frames in the sequence, so that the inputted sequence lengths capture more variance. In this example, during training, feature maps can be learned in a supervised manner by using manually annotated feature sets or features sets extracted using image processing methods such as Scale-Invariant Feature Transform "SIFT", Speeded Up Robust Features "SURF" as ground truth. Feature maps can also be learned in an unsupervised manner using neural networks such as auto encoders to learn a fixed size feature representation.

In another example, neural network 130 may include a CNN and an RNN. In this example, each frame in the temporal sequences of the subset of the sub-regions 120_{i,j} that are identified from the differential images in operation S130, is inputted into the CNN, and a low-dimensional representation of the frame is generated, for example a 1D feature vector. This feature vector is then inputted into an RNN, such as an LSTM or a Gated Recurrent Unit "GRU" to capture the temporal aspect of the input.

As mentioned above, in some examples the differential images are computed for a current image using an earlier image in the sequence, and wherein the time period ΔT between the generation of the current image and the generation of the earlier image in the sequence, is predetermined, such that each differential image represents a rate of change in image intensity values between the current image and the earlier image in the sequence. A benefit of inputting differential images that represent a rate of change in image intensity values between the current image and the earlier image in the sequence, into the neural network, in contrast to e.g. inputting differential images that represent e.g. DSA image data into the neural network, is that the former provides relatively higher emphasis on image frames representing dynamic changes in contrast agent flow and relatively lower emphasis on image frames representing continuous contrast agent flow, or no flow at all. In other words, it provides relatively higher emphasis on image frames Fr2, Fr3, Fr10 and Fr11 in Fig. 5 and Fig. 6. When contrast agent enters a region, the time derivative will be positive, and when contrast agent leaves an area the time derivative will be negative. This improves the efficiency of locating sub-regions of interest. In some examples, the images may be further processed by low-pass filtering in order to reduce the motion from e.g. cardiac and respiratory sources, patient movement, and noise. In some examples, the images can also be registered to each other, using e.g. rigid, affine, or a deformable registration, prior to imputing the temporal sequences of the sub-regions 120_{i,j} into the neural network in operation S 140, in order to further reduce the effect of motion.

In some examples, additional information is inputted into the neural network 130 in the form of a first arrival time for each sub-region of the vasculature. The first arrival time represents a time at which the contrast agent enters the sub-region. In these examples the method of locating a vascular constriction in a temporal sequence of angiographic images, includes:
computing a first arrival time for each sub-region of the vasculature representing a time at which the contrast agent enters the sub-region;
and wherein the neural network 130 is trained to classify a sub-region of the vasculature as including a vascular constriction from the temporal sequences of the vasculature and from the first arrival time;
and wherein the inputting the identified temporal sequences of the subset into a neural network 130 further comprises inputting the first arrival time of the sub-region into the neural network 130.

The first arrival time may be computed as the absolute time of the contrast agent entering each sub-region, or as the time difference with respect to another reference, such as between contrast agent entering a predetermined region of the vasculature, and the contrast agent entering each sub-region. For example, if the angiographic images represent the brain, the first arrival time may be determined as the time difference between contrast agent entering the base of the arterial tree, and the contrast agent entering each sub-region. The times of these events may, for example, be determined by segmenting the vasculature and applying a threshold to the sub-regions in order to detect the contrast agent. The first arrival time may be represented in a displayed temporal sequences of angiographic images of the vasculature, for example by color-coding the first arrival time in the displayed temporal sequence.

In some examples, additional information is inputted into the neural network in the form of corresponding image data from other imaging modalities. For example perfusion CT image data, CT angiography image data, or image data from other imaging modalities may be registered to the received temporal sequence of angiographic images 110 representing a flow of a contrast agent within a vasculature, and inputted into the neural network.

Additional information that is inputted into the neural network 130 in this manner, may assist the neural network in classifying a sub-region 120_{i,j} of the vasculature as including a vascular constriction 140.

Fig. 8 is a flowchart illustrating a method of training a neural network to locate a vascular constriction in a temporal sequence of angiographic images, in accordance with some aspects of the disclosure. The method may be used to train the neural networks described above. With reference to Fig. 8, a computer implemented method of training a neural network 130 to locate a vascular constriction in a temporal sequence of angiographic images, includes:
receiving S210 angiographic image training data including a plurality of temporal sequences of angiographic images representing a flow of contrast agent within a plurality of sub-regions of a vasculature; each temporal sequence being classified with a ground truth classification as including a vascular constriction or classified as not including a vascular constriction;
inputting S220 the received angiographic image training data into the neural network 130; and adjusting parameters of the neural network 130 based on a difference between the classification of each inputted temporal sequence generated by the neural network 130, and the ground truth classification.

The angiographic image training data may for example be provided by fluoroscopic or DSA imaging datasets. The training data may be provided in the form of differential images as described above. The training data may originate from one or more clinical sites. The training data may be collected from one or more subjects. The sub-regions of the angiographic image training data may be defined using the techniques described above so as to provide temporal sequences of the sub-regions 120_{i,j} which are inputted into the neural network in the operation S220. The ground truth classification for the sub-regions may be provided by an expert annotating the angiographic image training data with the location of any stenosis, or alternatively the absence of any stenosis.

**In** the training method described with reference to Fig. 8, parameters of the neural network 130 are adjusted automatically based on a difference between the classification of each inputted temporal sequence generated by the neural network 130, and the ground truth classification. The parameters that are adjusted in this procedure include the weights and the biases of activation functions in the neural network. The parameters are adjusted by inputting the training data, and computing the value of a loss function representing the difference between the classification of each inputted temporal sequence generated by the neural network 130, and the ground truth classification. Training is typically terminated when the neural network accurately provides the corresponding expected output data. The value of the loss function, or the error, may be computed using functions such as the negative log-likelihood loss, the mean squared error, or the Huber loss, or the cross entropy. During training, the value of the loss function is typically minimized, and training is terminated when the value of the loss function satisfies a stopping criterion. Sometimes, training is terminated when the value of the loss function satisfies one or more of multiple criteria.

Various methods are known for solving the loss minimization problem such as gradient descent, Quasi-Newton methods, and so forth. Various algorithms have been developed to implement these methods and their variants including but not limited to Stochastic Gradient Descent "SGD", batch gradient descent, mini-batch gradient descent, Gauss-Newton, Levenberg Marquardt, Momentum, Adam, Nadam, Adagrad, Adadelta, RMSProp, and Adamax "optimizers" These algorithms compute the derivative of the loss function with respect to the model parameters using the chain rule. This process is called backpropagation since derivatives are computed starting at the last layer or output layer, moving toward the first layer or input layer. These derivatives inform the algorithm how the model parameters must be adjusted in order to minimize the error function. That is, adjustments to model parameters are made starting from the output layer and working backwards in the network until the input layer is reached. In a first training iteration, the initial weights and biases are often randomized. The neural network then predicts the output data, which is likewise, random. Backpropagation is then used to adjust the weights and the biases. The training process is performed iteratively by making adjustments to the weights and biases in each iteration. Training is terminated when the error, or difference between the predicted output data and the expected output data, is within an acceptable range for the training data, or for some validation data. Subsequently the neural network may be deployed, and the trained neural network makes predictions on new input data using the trained values of its parameters. If the training process was successful, the trained neural network accurately predicts the expected output data from the new input data.

Training a neural network typically involves inputting a large training dataset into the neural network, and iteratively adjusting the neural network parameters until the trained neural network provides an accurate output. Training is often performed using a Graphics Processing Unit "GPU" or a dedicated neural processor such as a Neural Processing Unit "NPU" or a Tensor Processing Unit "TPU". Training therefore typically employs a centralized approach wherein cloud-based or mainframe-based neural processors are used to train a neural network. Following its training with the training dataset, the trained neural network may be deployed to a device for analyzing new input data; a process termed "inference". The processing requirements during inference are significantly less than those required during training, allowing the neural network to be deployed to a variety of systems such as laptop computers, tablets, mobile phones and so forth. Inference may for example be performed by a Central Processing Unit "CPU", a GPU, an NPU, a TPU, on a server, or in the cloud.

Fig. 9 is a schematic diagram illustrating a system 300 for locating a vascular constriction in a temporal sequence of angiographic images, in accordance with some aspects of the disclosure. The system 300 includes one or more processors 310 that are configured to perform one or more aspects of the above-described method. The system 300 may also include an X-ray imaging system 320, as illustrated in Fig. 3, and which may be configured to provide a temporal sequence of angiographic images 110 representing a flow of a contrast agent within a vasculature, for use in the above methods. The system 300 may also include a display 200 and/or a user interface device such as a keyboard, and/or a pointing device such as a mouse for controlling the execution of the method, and/or a patient bed 330. These items may be in communication with each other via wired or wireless communication, as illustrated in Fig. 9.

## Claims

1. A computer-implemented method of locating a vascular constriction in a temporal sequence of angiographic images, comprising:
receiving (S110) a temporal sequence of angiographic images (110) representing a flow of a contrast agent within a vasculature;
for at least some of the angiographic images in the temporal sequence, computing (S120) a differential image representing a difference in image intensity values between a current image and an earlier image in the sequence in a plurality of sub-regions (120_{i,j}) of the vasculature;
**characterised by**:
identifying (S130), from the differential images, temporal sequences of a subset of the sub-regions (120_{i,j}) and in which subset the contrast agent enters the sub-region, and the contrast agent subsequently leaves the sub-region;
inputting (S140) the identified temporal sequences of the subset into a neural network (130) trained to classify, from temporal sequences of angiographic images of the vasculature, a sub-region (120_{i,j}) of the vasculature as including a vascular constriction (140); and
identifying (S150), a sub-region that includes the vascular constriction (140) based on the classification provided by the neural network (130).

2. The computer-implemented method according to claim 1, wherein the neural network (130) is trained to classify, from temporal sequences of angiographic images of the vasculature, a sub-region (120_{i,j}) of the vasculature as including a vascular constriction (140), by:
receiving (S210) angiographic image training data including a plurality of temporal sequences of angiographic images representing a flow of a contrast agent within a plurality of sub-regions of a vasculature; each temporal sequence being classified with a ground truth classification identifying the temporal sequence as including a vascular constriction or not including a vascular constriction;
inputting (S220) the received angiographic image training data into the neural network (130); and adjusting parameters of the neural network (130) based on a difference between the classification of each inputted temporal sequence generated by the neural network (130), and the ground truth classification.

3. The computer-implemented method according to claim 1, wherein a time period (ΔT) between the generation of the current image and the generation of the earlier image in the sequence, that is used to compute each differential image, is predetermined, such that each differential image represents a rate of change in image intensity values between the current image and the earlier image in the sequence.

4. The computer-implemented method according to claim 1, wherein the earlier image is provided by a mask image, and wherein the same mask image is used to compute each differential image.

5. The computer-implemented method according to claim 1, wherein the identifying (S150), a sub-region that includes the vascular constriction (140), comprises: displaying a temporal sequence of angiographic images (110) representing a flow of a contrast agent within the identified sub-region that includes the vascular constriction (140), or displaying a temporal sequence of differential images representing a flow of a contrast agent within the identified sub-region that includes the vascular constriction (140).

6. The computer-implemented method according to claim 1, wherein the identifying (S130) temporal sequences of a subset of the sub-regions (120_{i,j}), comprises: identifying portions (Fr2..Fr11) of the temporal sequences generated between the contrast agent entering the sub-region and the contrast agent leaving the sub-region; and wherein the inputting the temporal sequences of the subset into a neural network (130), comprises inputting the identified portions (Fr2..Fr11) of the temporal sequences of the subset into the neural network (130).

7. The computer-implemented method according to claim 6, wherein the identifying (S130) temporal sequences of a subset of the sub-regions, comprises: identifying further portions (Fr4..Fr9) of the temporal sequences wherein the sub-region has a maximum amount of contrast agent, and excluding from the identified portions (Fr2..Fr11) of the temporal sequences the further portions (Fr4..Fr9) of the temporal sequences.

8. The computer-implemented method according to claim 1, comprising:
computing a first arrival time for each sub-region of the vasculature representing a time at which the contrast agent enters the sub-region;
and wherein the neural network (130) is trained to classify a sub-region of the vasculature as including a vascular constriction from the temporal sequences of the vasculature and from the first arrival time;
and wherein the inputting the identified temporal sequences of the subset into a neural network (130) further comprises inputting the first arrival time of the sub-region into the neural network (130).

9. The computer-implemented method according to claim 1, comprising defining the sub-regions of the vasculature by dividing the angiographic images in the received temporal sequence into a plurality of predefined sub-regions.

10. The computer-implemented method according to claim 1, comprising defining the sub-regions of the vasculature by segmenting the vasculature in the angiographic images and defining a plurality of sub-regions that overlap the vasculature.

11. The computer-implemented method according to claim 10, comprising identifying a plurality of branches in the segmented vasculature, and for each branch, determining an amount of contrast agent along an axial length of the branch, and representing the sub-region as a two dimensional graph indicating the amount contrast agent plotted against the axial length of the branch.

12. The computer-implemented method according to claim 1, comprising stacking, in the time domain, the identified temporal sequences of the subset of the sub-regions, prior to the inputting the identified temporal sequences of the subset into a neural network (130).

13. A computer program product comprising instructions which when executed by one or more processors, cause the one or more processors to carry the method according to any one of claims 1 - 12.

14. A system (300) for locating a vascular constriction in a temporal sequence of angiographic images; the system comprising one or more processors (310) configured to perform the method according to any one of claims 1 - 12.

## Patentansprüche

1. Computerimplementiertes Verfahren zum Lokalisieren einer Gefäßverengung in einer zeitlichen Abfolge angiographischer Bilder, umfassend:
Empfangen (S110) einer zeitlichen Abfolge angiographischer Bilder (110), die einen Fluss eines Kontrastmittels innerhalb eines Gefäßsystems darstellen;
Berechnen (S120) eines Differenzbildes für zumindest einige der angiographischen Bilder in der zeitlichen Abfolge, das eine Differenz in den Bildintensitätswerten zwischen einem aktuellen Bild und einem früheren Bild in der Abfolge in einer Vielzahl von Unterbereichen (120_{i,j}) des Gefäßsystems darstellt;
**gekennzeichnet durch**:
Identifizieren (S130) aus den Differenzbildern zeitlicher Abfolgen einer Teilmenge der Unterbereiche (120_{i,j}) und in welcher Teilmenge das Kontrastmittel in den Unterbereich eintritt und das Kontrastmittel anschließend den Unterbereich verlässt;
Eingeben (S140) der identifizierten zeitlichen Abfolgen der Teilmenge in ein neuronales Netzwerk (130), das trainiert ist, um aus zeitlichen Abfolgen angiographischer Bilder des Gefäßsystems einen Unterbereich (120_{i,j}) des Gefäßsystems als eine Gefäßverengung (140) beinhaltend zu klassifizieren; und
Identifizieren (S150) eines Unterbereichs, der die Gefäßverengung (140) beinhaltet, basierend auf der vom neuronalen Netzwerk (130) bereitgestellten Klassifizierung.

2. Computerimplementiertes Verfahren nach Anspruch 1, wobei das neuronale Netzwerk (130) trainiert ist, um aus zeitlichen Abfolgen angiographischer Bilder des Gefäßsystems einen Unterbereich (120_{i,j}) des Gefäßsystems als eine Gefäßverengung (140) beinhaltend zu klassifizieren, durch:
Empfangen (S210) von angiographischen Bild-Trainingsdaten, die eine Vielzahl von zeitlichen Abfolgen angiographischer Bilder beinhalten, die einen Fluss eines Kontrastmittels innerhalb einer Vielzahl von Unterbereichen eines Gefäßsystems darstellen; wobei jede zeitliche Abfolge mit einer Ground-Truth-Klassifizierung klassifiziert wird, welche die zeitliche Abfolge als eine Gefäßverengung beinhaltend oder keine Gefäßverengung beinhaltend identifiziert;
Eingeben (S220) der empfangenen angiographischen Bild-Trainingsdaten in das neuronale Netzwerk (130); und Anpassen von Parametern des neuronalen Netzwerks (130) basierend auf einer Differenz zwischen der Klassifizierung jeder eingegebenen zeitlichen Abfolge, die durch das neuronale Netzwerk (130) erzeugt wird, und der Ground-Truth-Klassifizierung.

3. Computerimplementiertes Verfahren nach Anspruch 1, wobei ein Zeitraum (ΔT) zwischen der Erzeugung des aktuellen Bildes und der Erzeugung des früheren Bildes in der Abfolge, der verwendet wird, um jedes Differenzbild zu berechnen, vorbestimmt ist, sodass jedes Differenzbild eine Änderungsrate der Bildintensitätswerte zwischen dem aktuellen Bild und dem früheren Bild in der Abfolge darstellt.

4. Computerimplementiertes Verfahren nach Anspruch 1, wobei das frühere Bild durch ein Maskenbild bereitgestellt wird, und wobei dasselbe Maskenbild verwendet wird, um jedes Differenzbild zu berechnen.

5. Computerimplementiertes Verfahren nach Anspruch 1, wobei das Identifizieren (S150) eines Unterbereichs, der die Gefäßverengung (140) beinhaltet, umfasst: Anzeigen einer zeitlichen Abfolge angiographischer Bilder (110), die einen Fluss eines Kontrastmittels innerhalb des identifizierten Unterbereichs darstellen, der die Gefäßverengung (140) beinhaltet, oder Anzeigen einer zeitlichen Abfolge von Differenzbildern, die einen Fluss eines Kontrastmittels innerhalb des identifizierten Unterbereichs darstellen, der die Gefäßverengung (140) beinhaltet.

6. Computerimplementiertes Verfahren nach Anspruch 1, wobei das Identifizieren (S130) zeitlicher Abfolgen einer Teilmenge der Unterbereiche (120_{i,j}) umfasst: Identifizieren von Abschnitten (Fr2..Fr11) der zeitlichen Abfolgen, die zwischen dem Eintritt des Kontrastmittels in den Unterbereich und dem Verlassen des Unterbereichs durch das Kontrastmittel erzeugt werden; und wobei das Eingeben der zeitlichen Abfolgen der Teilmenge in ein neuronales Netzwerk (130) Eingeben der identifizierten Abschnitte (Fr2..Fr11) der zeitlichen Abfolgen der Teilmenge in das neuronale Netzwerk (130) umfasst.

7. Computerimplementiertes Verfahren nach Anspruch 6, wobei das Identifizieren (S130) zeitlicher Abfolgen einer Teilmenge der Unterbereiche umfasst: Identifizieren weiterer Abschnitte (Fr4..Fr9) der zeitlichen Abfolgen, in denen der Unterbereich eine maximale Kontrastmittelmenge aufweist, und Ausschließen der weiteren Abschnitte (Fr4..Fr9) der zeitlichen Abfolgen aus den identifizierten Abschnitten (Fr2..Fr11) der zeitlichen Abfolgen.

8. Computerimplementiertes Verfahren nach Anspruch 1, umfassend:
Berechnen einer ersten Ankunftszeit für jeden Unterbereich des Gefäßsystems, die einen Zeitpunkt darstellt, zu dem das Kontrastmittel in den Unterbereich eintritt;
und wobei das neuronale Netzwerk (130) trainiert ist, um aus den zeitlichen Abfolgen des Gefäßsystems und aus der ersten Ankunftszeit einen Unterbereich des Gefäßsystems als eine Gefäßverengung beinhaltend zu klassifizieren;
und wobei das Eingeben der identifizierten zeitlichen Abfolgen der Teilmenge in ein neuronales Netzwerk (130) weiter Eingeben der ersten Ankunftszeit des Unterbereichs in das neuronale Netzwerk (130) umfasst.

9. Computerimplementiertes Verfahren nach Anspruch 1, welches Definieren der Unterbereiche des Gefäßsystems durch Unterteilen der angiographischen Bilder in der empfangenen zeitlichen Abfolge in eine Vielzahl vordefinierter Unterbereiche umfasst.

10. Computerimplementiertes Verfahren nach Anspruch 1, welches Definieren der Unterbereiche des Gefäßsystems durch Segmentieren des Gefäßsystems in den angiographischen Bildern und Definieren einer Vielzahl von Unterbereichen, die das Gefäßsystem überlappen, umfasst.

11. Computerimplementiertes Verfahren nach Anspruch 10, welches Identifizieren einer Vielzahl von Verzweigungen im segmentierten Gefäßsystem, und für jede Verzweigung Bestimmen einer Menge an Kontrastmittel entlang einer axialen Länge der Verzweigung, und Darstellen des Unterbereichs als zweidimensionalen Graphen, der die Menge an Kontrastmittel angibt, die über der axiale Länge der Verzweigung aufgetragen ist, umfasst.

12. Computerimplementiertes Verfahren nach Anspruch 1, welches Stapeln im Zeitbereich der identifizierten zeitlichen Abfolgen der Teilmenge der Unterbereiche vor dem Eingeben der identifizierten zeitlichen Abfolgen der Teilmenge in ein neuronales Netzwerk (130) umfasst.

13. Computerprogrammprodukt, das Anweisungen umfasst, die, wenn sie von einem oder mehreren Prozessoren ausgeführt werden, den einen oder mehrere Prozessoren veranlassen, das Verfahren nach einem der Ansprüche 1 - 12 auszuführen.

14. System (300) zum Lokalisieren einer Gefäßverengung in einer zeitlichen Abfolge angiographischer Bilder; wobei das System einen oder mehrere Prozessoren (310) umfasst, die konfiguriert sind, um das Verfahren nach einem der Ansprüche 1 - 12 durchzuführen.

## Revendications

1. Procédé mis en œuvre par ordinateur de localisation d'une constriction vasculaire dans une séquence temporelle d'images angiographiques, comprenant :
la réception (S110) d'une séquence temporelle d'images angiographiques (110) représentant un écoulement d'un agent de contraste dans un système vasculaire ;
pour au moins certaines des images angiographiques dans la séquence temporelle, le calcul (S120) d'une image différentielle représentant une différence de valeurs d'intensité d'image entre une image actuelle et une image antérieure dans la séquence dans une pluralité de sous-régions (120_{i,j}) du système vasculaire ;
**caractérisé par** :
l'identification (S130), à partir des images différentielles, de séquences temporelles d'un sous-ensemble des sous-régions (120_{i,j}) et dans quel sous-ensemble l'agent de contraste entre dans la sous-région, et l'agent de contraste sort ensuite de la sous-région ;
l'entrée (S140) des séquences temporelles identifiées du sous-ensemble dans un réseau neuronal (130) entraîné pour classer, à partir de séquences temporelles d'images angiographiques du système vasculaire, une sous-région (120_{i,j}) du système vasculaire comme incluant une constriction vasculaire (140) ; et
l'identification (S150) d'une sous-région qui inclut la constriction vasculaire (140) sur la base de la classification fournie par le réseau neuronal (130).

2. Procédé mis en œuvre par ordinateur selon la revendication 1, dans lequel le réseau neuronal (130) est entraîné pour classer, à partir de séquences temporelles d'images angiographiques du système vasculaire, une sous-région (120_{i,j}) du système vasculaire comme incluant une constriction vasculaire (140), par :
la réception (S210) de données d'entraînement d'images angiographiques incluant une pluralité de séquences temporelles d'images angiographiques représentant un écoulement d'un agent de contraste dans une pluralité de sous-régions d'un système vasculaire ; chaque séquence temporelle étant classée avec une classification de vérité fondamentale identifiant la séquence temporelle comme incluant une constriction vasculaire ou n'incluant pas de constriction vasculaire ;
l'entrée (S220) des données d'entraînement d'images angiographiques reçues dans le réseau neuronal (130) ; et l'ajustement des paramètres du réseau neuronal (130) sur la base d'une différence entre la classification de chaque séquence temporelle entrée générée par le réseau neuronal (130) et la classification de vérité fondamentale.

3. Procédé mis en œuvre par ordinateur selon la revendication 1, dans lequel une période de temps (ΔT) entre la génération de l'image actuelle et la génération de l'image antérieure dans la séquence, qui est utilisée pour calculer chaque image différentielle, est prédéterminée, de telle sorte que chaque image différentielle représente un taux de changement de valeurs d'intensité d'image entre l'image actuelle et l'image antérieure dans la séquence.

4. Procédé mis en œuvre par ordinateur selon la revendication 1, dans lequel l'image antérieure est fournie par une image de masque et dans lequel la même image de masque est utilisée pour calculer chaque image différentielle.

5. Procédé mis en œuvre par ordinateur selon la revendication 1, dans lequel l'identification (S150) d'une sous-région qui inclut la constriction vasculaire (140) comprend : l'affichage d'une séquence temporelle d'images angiographiques (110) représentant un écoulement d'un agent de contraste dans la sous-région identifiée qui inclut la constriction vasculaire (140), ou l'affichage d'une séquence temporelle d'images différentielles représentant un écoulement d'un agent de contraste dans la sous-région identifiée qui inclut la constriction vasculaire (140).

6. Procédé mis en œuvre par ordinateur selon la revendication 1, dans lequel l'identification (S130) de séquences temporelles d'un sous-ensemble des sous-régions (120_{i,j}), comprend : l'identification de parties (Fr2..Fr11) des séquences temporelles générées entre l'agent de contraste entrant dans la sous-région et l'agent de contraste sortant de la sous-région ; et dans lequel l'entrée des séquences temporelles du sous-ensemble dans un réseau neuronal (130) comprend l'entrée des parties (Fr2..Fr11) identifiées des séquences temporelles du sous-ensemble dans le réseau neuronal (130).

7. Procédé mis en œuvre par ordinateur selon la revendication 6, dans lequel l'identification (S130) de séquences temporelles d'un sous-ensemble des sous-régions comprend : l'identification de parties supplémentaires (Fr4..Fr9) des séquences temporelles, dans lequel la sous-région présente une quantité maximale d'agent de contraste, et l'exclusion des parties supplémentaires (Fr4..Fr9) des séquences temporelles hors des parties identifiées (Fr2..Fr11) des séquences temporelles.

8. Procédé mis en œuvre par ordinateur selon la revendication 1, comprenant :
le calcul d'un premier temps d'arrivée pour chaque sous-région du système vasculaire représentant un moment auquel l'agent de contraste entre dans la sous-région ;
et dans lequel le réseau neuronal (130) est entraîner pour classer une sous-région du système vasculaire comme incluant une constriction vasculaire à partir des séquences temporelles du système vasculaire et à partir du premier temps d'arrivée ;
et dans lequel l'entrée des séquences temporelles identifiées du sous-ensemble dans un réseau neuronal (130) comprend en outre l'entrée du premier temps d'arrivée de la sous-région dans le réseau neuronal (130).

9. Procédé mis en œuvre par ordinateur selon la revendication 1, comprenant la définition des sous-régions du système vasculaire en divisant les images angiographiques dans la séquence temporelle reçue en une pluralité de sous-régions prédéfinies.

10. Procédé mis en œuvre par ordinateur selon la revendication 1, comprenant la définition des sous-régions du système vasculaire en segmentant le système vasculaire dans les images angiographiques et la définition d'une pluralité de sous-régions qui chevauchent le système vasculaire.

11. Procédé mis en œuvre par ordinateur selon la revendication 10, comprenant l'identification d'une pluralité de branches dans le système vasculaire segmenté et, pour chaque branche, la détermination d'une quantité d'agent de contraste le long d'une longueur axiale de la branche et la représentation de la sous-région sous la forme d'un graphique bidimensionnel indiquant la quantité d'agent de contraste tracée en fonction de la longueur axiale de la branche.

12. Procédé mis en œuvre par ordinateur selon la revendication 1, comprenant l'empilement, dans le domaine temporel, des séquences temporelles identifiées du sous-ensemble des sous-régions, avant l'entrée des séquences temporelles identifiées du sous-ensemble dans un réseau neuronal (130).

13. Produit de programme informatique comprenant des instructions qui, lorsqu'elles sont exécutées par un ou plusieurs processeurs, amènent les un ou plusieurs processeurs à effectuer le procédé selon l'une quelconque des revendications 1-12.

14. Système (300) permettant de localiser une constriction vasculaire dans une séquence temporelle d'images angiographiques ; le système comprenant un ou plusieurs processeurs (310) configurés pour réaliser le procédé selon l'une quelconque des revendications 1-12.
